(19) Europäisches Patentamt
European Patent Office
Office européen des brevets

(11) EP 0 916 650 B1

(12) **FASCICULE DE BREVET EUROPEEN**

(45) Date de publication et mention de la délivrance du brevet:
**09.01.2002 Bulletin 2002/02**

(51) Int Cl.7: **C07C 255/31**, C11B 9/00, C07C 47/225, C07C 47/11

(21) Numéro de dépôt: **98120349.0**

(22) Date de dépôt: **28.10.1998**

(54) **Nitriles et aldéhydes dérivés du 3-isopropényl-1,2-diméthyl-1-cyclopentanol et leur utilisation en parfumerie**

Von 3-Isopropenyl-1,2-dimethyl-1-cyclopentanol abgeleitete Nitrile und Aldehyde und ihre Verwendung in der Parfümerie

Nitriles and aldehydes derived from 3-isopropenyl-1,2-dimethyl-1-cyclopentanol and their use in perfumery

(84) Etats contractants désignés:
**CH DE ES FR GB IT LI NL**

(30) Priorité: **11.11.1997 CH 259297**

(43) Date de publication de la demande:
**19.05.1999 Bulletin 1999/20**

(73) Titulaire: **FIRMENICH SA**
**1211 Genève 8 (CH)**

(72) Inventeur: **Giersch, Wolfgang**
**1233 Bernex (CH)**

(74) Mandataire:
**Salvaterra-Garcia, Maria de Lurdes, Dr.**
**Firmenich SA Case Postale 239**
**1211 Genève 8 (CH)**

(56) Documents cités:
**DE-A- 2 447 170** **FR-A- 2 372 784**
**US-A- 4 168 281**

- **H. STRICKLER ET AL: "78. Zur Kenntnis der ätherischen Öle. Die thermiasche Cyclisation des (-)-R-Linalools." HELVETICA CHIMICA ACTA., vol. 50, 1967, pages 759-797, XP002102683 BASEL CH**
- **CHALK, A. J.: "Hydroformylation of terpenes and related molecules" CHEM. IND. (DEKKER) (1988), 33(CATAL. ORG. REACT.), 43-63 , XP002102684**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 916 650 B1

## Description

La présente invention a trait au domaine de la parfumerie. Elle concerne plus particulièrement les composés de formule

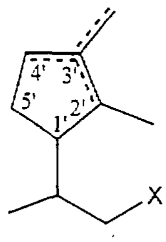

(I)

dans laquelle X représente un groupe C ≡ N ou un groupe C(H) = O et dans laquelle le cycle en $C_5$ est soit saturé, soit comprend une double liaison dans l'une des positions indiquées par les pointillés, ou tout mélange de deux ou plusieurs composés de formule (I), et leur utilisation en parfumerie.

Les composés de formule (I) sont nouveaux et leur utilisation en parfumerie n'a jamais été ni décrite, ni suggérée. Ils portent une fonction aldéhyde ou une fonction nitrile et tous ont une odeur caractéristique de type citron-vert qui est très appréciée en parfumerie.

L'invention comprend d'une part des composés de formule (I) dans laquelle Y est un groupe C ≡ N, c'est-à-dire appartenant à la famille chimique des nitriles, qui sont particulièrement appropriés pour un emploi dans des milieux agressifs. Elle comprend d'autre part les aldéhydes correspondants, de structure chimique similaire, qui montrent le même type d'odeur que les nitriles.

Il faut noter que l'on connaît dans l'industrie de la parfumerie un grand nombre de composés appartenant à la famille chimique des nitriles. Dans le contexte de la présente invention, il convient de citer les nitriles ci-après d'un usage courant en parfumerie, en particulier en parfumerie fonctionnelle.

Le géranyle nitrile (3,7-diméthyl-2,6-octadiènenitrile) possède une odeur puissante verte et chimique qui ressemble à celle du citral (Z-3,7-diméthyl-2,6-octadiénal), ce dernier étant lui-même un composé très répandu dans des applications de parfumerie et se trouvant dans la nature.

Le citronellyl nitrile (3,7-diméthyl-6-octènenitrile), montre lui une note olfactive qui rappelle l'odeur du citron, avec une sous-note caractéristique des nitriles. La note hespéridée est de même très prononcée chez l'Ozonil® (mélange de 2-tridécènenitrile et 3-tridécènenitrile; origine : Haarmann & Reimer, Allemagne), mais là on trouve aussi des notes de type mandarine-fruitées, pêche qui sont associées à une sous-note florale.

Enfin, le citronitrile (3-méthyl-5-phényl-2-pentènenitrile) présente une odeur du même type que celle des composés mentionnés ci-dessus, à savoir hespéridée-fruitée.

Les détergents, les déodorants ou les antiperspirants et les savons sont des exemples de milieux agressifs dans lesquels le citral, par exemple, que l'on peut considérer comme un composé représentatif des odeurs de type hespéridé, avec sa puissante note hespéridée-verte, se montre instable, ce qui empêche son utilisation dans le domaine de la parfumerie fonctionnelle, et cela malgré son odeur très prisée par les parfumeurs.

Or, bien que montrant des similarités olfactives avec le citral, les nitriles connus présentés plus haut ne possèdent pas la qualité olfactive de celui-ci. Leurs notes sont moins caractéristiques du citron, moins fraîches-hespéridées, et on trouve dans tous ces nitriles une connotation grasse-métallique. Pour ces raisons, la recherche de nitriles qui soient stables dans les milieux agressifs et qui possèdent une odeur proche de la note hespéridée-verte du type de l'odeur du citral, reste une tâche d'actualité.

La présente invention apporte justement une solution originale à ce problème grâce à de nouveaux composés ayant cette odeur désirée.

En effet, nous avons trouvé que les composés de formule

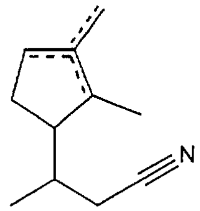

(I')

en particulier, ayant une double liaison dans une des positions indiquées par les lignes pointillées, ou tout mélange de deux ou plusieurs de ces composés, possédaient non seulement une excellente stabilité dans des milieux agressifs, mais aussi une odeur qui est très semblable à celle du citral.

Ainsi, l'odeur des composés de formule (I'), ou de leurs mélanges, possède le même côté frais-hespéridé que le citral, ce qui est très inattendu pour un nitrile. On note également une connotation verte qui évoque la limette, et les composés de l'invention ne possèdent pas la connotation grasse-métallique que l'on trouve généralement chez les nitriles d'usage courant en parfumerie (voir plus haut pour des exemples courants). D'une façon générale, l'odeur des composés de l'invention est très fraîche et propre.

On préfère, selon l'invention, utiliser en tant qu'ingrédient parfumant un mélange de composés de formule (I') contenant au moins 30% en poids d'un composé de formule

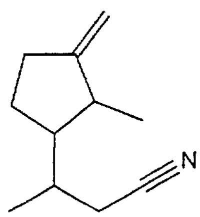

(I'a)

Encore plus appréciés sont les mélanges mentionnés auparavant qui contiennent, outre le composé (I'a) dans la quantité précisée, environ 60% en poids du composé de formule

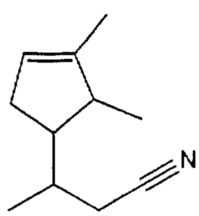

(I'b)

portant une double liaison endocyclique en position 3' du cycle.

Selon un mode d'exécution encore plus apprécié, on utilise un composé de formule (I'a) à l'état pur, dans lequel se trouve représenté au mieux le caractère frais-hespéridé, vert-limette du citral.

Il s'est avéré, cependant, que les caractéristiques olfactives mentionnées ci-dessus sont également présentes dans les mélanges de l'invention mentionnés plus haut, d'une façon qui satisfait les critères du parfumeur, ce qui rend également possible l'utilisation desdits mélanges en tant qu'ingrédients parfumants. L'avantage de l'utilisation de ces mélanges par rapport à celle de leurs ingrédients à l'état pur est de nature économique, vu que ces mélanges peuvent être obtenus directement de la synthèse décrite ci-après, sans qu'il soit nécessaire d'utiliser des techniques de séparation particulières.

Les nitriles de la présente invention sont accessibles par une synthèse à plusieurs étapes qui utilise le 3-isopropényl-1,2-diméthyl-1-cyclopentanol, ou l'un de ses isomères optiquement actifs, en tant que produit de départ. Cet alcool cyclique est un composé qui est commercialement disponible et qui se forme lors de la pyrolyse du (-)-R-linalool, cette réaction menant à un mélange de quatre diastéréomères (voir H. Strickler, G. Ohloff et E.sz.Kovàts, Helv. Chim. Acta 1967, *50*, 759).

Le schéma ci-dessous montre la synthèse que nous avons développée pour les nitriles de formule (I):

Schéma I

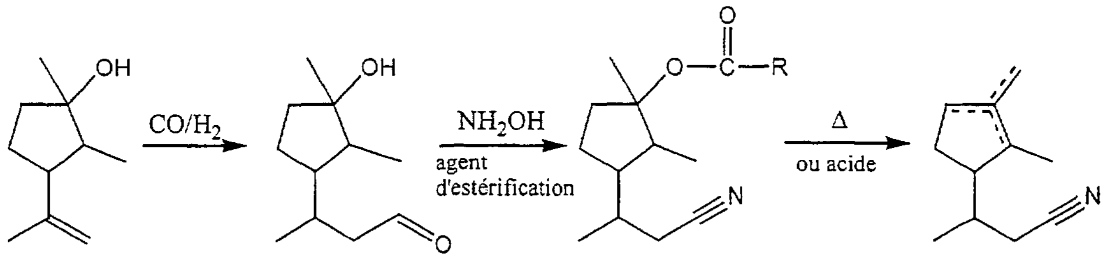


(II) (I')

R = alkyle ou aryle

La première étape de cette synthèse comprend l'hydroformylation de l'alcool cyclique de départ pour former l'aldéhyde correspondant. La réaction d'hydroformylation étant connue de l'homme de métier, il est à même de choisir les conditions appropriées de réaction, par exemple choix du catalyseur, solvant, pression, qui mènent aux bons résultats, sans qu'il soit nécessaire d'une description plus détaillée.

L'aldéhyde ainsi obtenu est ensuite transformé en nitrile correspondant, en utilisant, par exemple, de l'hydroxylamine, ou l'un de ses sels, en présence d'une base. Dans l'étape suivante, le composé obtenu est estérifié à l'aide d'un agent d'estérification courant, tel que l'anhydride acétique. Il s'est avéré avantageux de ne pas isoler le nitrile mentionné ci-dessus du milieu réactionnel mais de l'estérifier directement dans le même milieu.

La dernière étape du procédé est la réaction d'élimination de l'ester (II) ainsi obtenu, de préférence de l'acétate de 3-(2'-cyano-1-méthyléthyl)-1,2-diméthyl-1-cyclopentyle, qui mène au produit final souhaité, à savoir les nitriles insaturés de formules (I'a) et (I'b). L'élimination peut se dérouler sous des conditions variées qui sont courantes pour l'homme du métier. Il convient de mentionner, dans ce contexte, l'emploi d'un acide, comme par exemple l'acide p-toluènesulfonique pour effectuer la réaction d'élimination.

Nous avons, cependant, obtenu les meilleurs résultats en ayant recours à un traitement thermique, à savoir une pyrolyse de l'ester nitrile, cette réaction donnant un produit brut qui contient une quantité olfactivement satisfaisante du nitrile le plus souhaité (I'a), tandis que l'élimination à l'aide d'un acide favorise souvent la formation des nitriles ayant des doubles liaisons en position endocyclique, à savoir les nitriles de formules (I'b) et (I'c) ci-dessous :

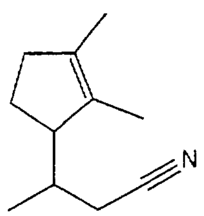

(I'c)

moins appréciés d'un point de vue olfactif.

Nous avons trouvé que, lorsque la pyrolyse se déroulait dans une plage de températures allant d'environ 400 à 550°C, de préférence d'environ 430 à 500°C, et sous une pression d'environ 10 à $50x10^2$ Pa, on pouvait obtenir des produits qui contenaient environ 30% en poids du nitrile selon la formule (I'a). Ces mélanges contiennent, hors de ce composé (I'a), les nitriles correspondant aux formules (I'b) et (I'c), dont la répartition varie selon les conditions employées.

Lesdits mélanges peuvent être utilisés tels quels en tant qu'ingrédients parfumants, car ils ont tous les caractéristiques olfactives du nitrile (I'a), c'est-à-dire la note fraîche-hespéridée, verte-limette typique, l'impression globale étant pratiquement aussi bonne que celle du nitrile pur.

Bien entendu, il est aussi possible d'utiliser des mélanges du type décrit auparavant qui contiennent des quantités du nitrile (I'a) qui sont différentes de la quantité mentionnée, (30% en poids), à condition qu'il soit présent en quantité effective, c'est-à-dire en quantité suffisante pour faire ressortir le caractère olfactif du nitrile mentionné. Cependant, nous avons constaté que les mélanges contenant 30% ou plus de ce nitrile étaient préférés selon l'invention.

Bien entendu, étant donné que la pyrolyse du (-)-(R)-linalol fournit un mélange de quatre diastéréomères qui peuvent être isolés par des techniques courantes, chacun de ces diastéréomères peut ensuite être utilisé séparément pour préparer les nitriles désirés, la stéréochimie de ces derniers étant alors déterminée par celle du diastéréomère de départ utilisé, qui demeure inchangée pendant toute la synthèse décrite plus haut. Ceci deviendra plus évident à l'étude des exemples qui suivront. Selon leur stéréochimie, l'odeur des composés obéissant à la formule (I'a) et ayant une stéréochimie définie peut être différente de celle d'un mélange de diastéréomères.

On a découvert que la meilleure qualité olfactive est atteinte lorsque le cyclopentanol de départ a la configuration (1R,2S,3S). On obtient alors un mélange composé de nitriles de formule (I'a) de configuration (1'R,2'S,3S) et (1'R,2'S,3R) et de nitriles de formule (I'b) de configuration (1'R, 2'S,3R) et (1'R,2'S,3S). Ces mélanges, et en particulier les stéréoisomères de formule (I'a) décrits ci-dessus, sont préférés selon l'invention.

On a obtenu un mélange de nitriles de qualité quasi similaire à partir d'un cyclopentanol de configuration (1R, 2R,3R), conduisant à un mélange final, d'une part de nitriles de formule (I'a) de configuration (1'S,2'R), et d'autre part de nitriles de formule (I'b) de configuration (1'S, 2'R).

La qualité olfactive du mélange obtenu à partir du cyclopentanol de configuration (1R,2S,3R), c'est-à-dire du mélange de nitriles de formule (I'a) ayant une configuration (1'S,2'S) et de nitriles de formule (I'b) ayant une configuration (1'S,2'S), est inférieure à la qualité des nitriles obtenus plus haut. Ici, la note citronnée est plus légère et des connotations graisseuses et poudreuses se perçoivent, le côté floral-hespéridé restant cependant clairement présent.

On a découvert que le 3-(2,3-diméthyl-1-cyclopentyl)butanenitrile de formule (I'd) (voir Schéma II ci-dessous), comportant un cycle saturé à cinq carbones, possédait également dans son odeur une note citron vert caractéristique ainsi qu'une légère connotation nitrile.

La présente invention concerne également les aldéhydes de formule (I") représentés ci-dessous comportant une double liaison dans l'une des positions indiquées par les pointillés, ou tout mélange de deux ou plus de ces composés. Le Schéma (II) suivant indique la synthèse de ces aldéhydes, ainsi que la synthèse des aldéhydes cycliques saturés correspondant de formule (I"a).

Schéma II

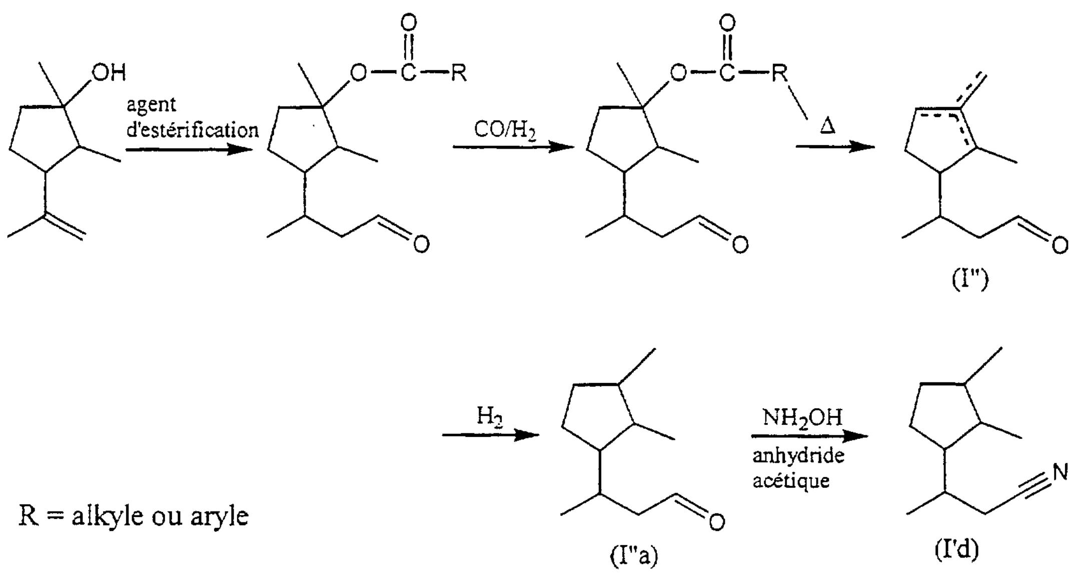


(I")

R = alkyle ou aryle

(I"a)

(I'd)

Les aldéhydes à cycle insaturé de formule (I") sont utiles dans le domaine de la parfumerie en raison de leur note de type frais-hespéridé et citon vert accompagnée d'une connotation typiquement aldéhydique. L'odeur caractéristique est proche de celle du 3,5,5-triméthylehéxanal (isononylaldéhyde), composé de large utilisation en parfumerie, capable de conférer des notes de type vert à une composition.

L'aldéhyde à cycle saturé correspondant (I"a) a une odeur de type vert-hespéridé, accompagnée d'une connotation terreuse et légèrement métallique, ainsi que d'une sous-note de type lemongrass.

Les nitriles de la présente invention se sont avérés complètement stables dans beaucoup de milieux typiquement agressifs pour les parfums. On peut citer dans ce contexte des détergents contenant des agents de blanchissage

et des activateurs tels que, par exemple, la tétracétyléthylènediamine (TAED), des hypohalites, hypochlorites en particulier, des agents de blanchissage peroxygénés tels que les perborates, etc. De plus, on peut citer les déodorants et antiperspirants corporels, qui contiennent par exemple des sels d'aluminium.

D'une façon générale, les nitriles de la présente invention sont employés de préférence dans des applications telles que savons, shampoings, déodorants et antiperspirants corporels, détergents liquides ou solides destinés au traitement de textiles, adoucissants textiles, ou encore compositions détergentes ou produits d'entretien destinés au nettoyage de la vaisselle ou de surfaces variées, qu'ils soient voués à un usage domestique ou industriel.

Bien entendu, l'utilisation des nitriles de la présente invention n'est cependant pas limitée aux produits mentionnés plus haut, et ces composés se prêtent également à tous les autres emplois courants en parfumerie, à savoir le parfumage de savons et gels de douche, de produits d'hygiène ou de traitement des cheveux, ainsi que de déodorants corporels, désodorisants d'air ambiant ou encore de préparations cosmétiques et même à l'emploi en parfumerie fine dans les parfums et eaux de toilette.

Les aldéhydes de la présente invention peuvent également faire l'objet de toutes les utilisations citées ci-dessus.

Dans ces applications, les composés de l'invention peuvent être utilisés seuls ou en mélange avec d'autres ingrédients parfumants, des solvants ou adjuvants d'usage courant en parfumerie. La nature et la variété de ces coingrédients n'appelle pas une description plus détaillée ici, qui ne saurait d'ailleurs être exhaustive, l'homme de métier étant à même de les choisir de par ses connaissances générales et en fonction de la nature du produit à parfumer et de l'effet olfactif recherché.

Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les produits divers susmentionnés varient dans une gamme de valeurs étendue. Ces valeurs sont dépendantes de la nature de l'article ou produit que l'on veut parfumer et de l'effet olfactif recherché, ainsi que de la nature des coingrédients dans une composition donnée lorsque les composés de l'invention sont utilisés en mélange avec des coingrédients parfumants, des solvants ou des adjuvants d'usage courant dans l'art.

A titre d'exemple, on peut citer des concentrations typiques de l'ordre de 0,1 à 10%, voire plus, en poids de ces composés, par rapport au poids de composition parfumante dans laquelle ils sont incorporés. Des concentrations bien inférieures à celles-ci peuvent être utilisées lorsque les composés sont directement appliqués dans le parfumage des produits de consommation divers cités auparavant.

L'invention sera maintenant décrite de façon plus détaillée dans les exemples suivants dans lesquels les abréviations ont le sens connu dans l'art. Les températures sont données en degrés Celsius, et les valeurs RMN (déplacement chimique δ) sont indiquées en ppm par rapport au TMS choisi comme standard.

Exemple 1

Préparation de nitriles insaturés selon l'invention

**A.** Hydroformylation du 3-isopropényl-1,2-diméthyl-1-cyclopentanol (procédé général pour tous les isomères)

Un autoclave a été chargé avec 900 g (5,4 mole) de 3-isopropényl-1,2- diméthyl-1-cyclopentanol, 63 g (0,24 mole ; origine : Glidco) de triphénylphosphine et 0,6 g (0,65 mmole) de $[(C_6H_5)_3P]_3Rh(CO)H$. L'autoclave a été fermé, pressurisé avec un mélange de $CO/H_2$ (1:1) jusqu'à $14x10^5$ Pa. On a chauffé à 110° et la réaction a été poursuivie pendant 5 jours, au bout desquels on a arrêté la réaction pour obtenir 920 g (conversion 60%) de produit brut.
Le mélange obtenu a été distillé à 102-105° ($6x10^1$ Pa) et on a isolé 400 g (2,17 mole) du 3-(3'-hydroxy-2',3'-diméthyl-1'-cyclopentyl)butanal.

Données spectrales de l'isomère de configuration (1'R,2'S,3'R), préparé à partir du (1R,2S,3S)-3-isopropényl-1,2-diméthyl-1-cyclopentanol:

RMN($^1$H): 0,88/0,90(deux d, J=7, 3H); 0,93(d, J=7, 3H); 0,97(d, J=6,5, 3H); 1,33(s, 3H); 9,78(large s, 1H) δ ppm
RMN($^{13}$C) :

(premier isomère) : 8,43 ; 18,59 ; 25,33 ; 29,86 ; 29,93 ; 37,7 ; 45,03 ; 46,86 ; 49,54 ; 80,04 ; 202,83 δ ppm
(deuxième isomère) : 8,75 ; 19,48 ; 25,59 ; 29,86 ; 30,27 ; 37,73 ; 45,03 ; 47,25 ; 49,94 ; 80,10 ; 203,22 δ ppm

SM :

(premier isomère) : 184(M+,1), 169(2), 166(2), 151(6), 123(11), 108(38), 95(22), 81(18), 71(98), 43(100)
(deuxième isomère) : 184(M+,0), 169(1), 166(4), 151(5), 123(13), 108(33), 95(32), 81(36), 71(97), 43(100)

**B.** Synthèse de l'acétate de 3-(2'-cyano-1'-méthyléthyl)-1,2-diméthyl-1-cyclopentyle (procédé général pour tous les isomères)

Le mélange de produits obtenu sous A. ci-dessus (202 g, 1,09 mole), $NH_2OH$-HCl (98 g, 1,4 mole) et KOH (91 g, 1,6 mole) dans le toluène (1,51) ont été chauffés à reflux pendant 150 minutes, en maintenant la solution sous agitation. Ensuite, on a refroidi le mélange jusqu'à température ambiante et lavé ce dernier à l'eau (400 ml), puis à la saumure (400 ml). On a repris la solution obtenue et on l'a chauffée à reflux pour distiller 500 ml de toluène. On a ensuite ajouté 800 g (7,84 mole) d'anhydride acétique, tout en maintenant la température du mélange. La réaction finie, le solvant a été enlevé par distillation avant de distiller le produit souhaité que l'on a obtenu avec un rendement de 84% (206 g) et qui est composé de deux diastéréomères. Point d'ébullition : 100° ($5x10^1$ Pa)
Données spectrales de l'isomère de configuration (1R,2S,3R), préparé à partir de (1'R,2'S,3'R)-3-(3'-hydroxy-2',3'-diméthyl-1'-cyclopentyl)butanal:

RMN($^1$H): 0,77/0,78(deux d, J=8, 3H); 1,1/1,12(deux d, J=6, 3H); 1,52(s, 3H) ; 2,01(s, 3H) δ ppm
RMN($^{13}$C) :

(premier isomère) : 8,93 ; 18,6 ; 23,51 ; 23,86 : 25,54 ; 32,52 ; 35,2 ; 43,49 ; 45,11 ; 88,68 ; 170,17 δ ppm
(deuxième isomère) : 8,74; 18,18; 23,34; 23,86; 25,47; 32,52; 35,11 ; 43,22 ; 44,99 ; 88,61 ; 170,17 δ ppm

SM:

(premier isomère): 223($M^+$,0), 183(3), 165(21), 152(12), 123(18), 110(23), 95(17), 71(20), 43(100)
(deuxième isomère) : 223($M^+$,0), 183(2), 165(26), 152(6), 123(20), 110(11), 95(20), 71(25), 43(100)

**C.** Pyrolyse de l'acétate de 3-(2'-cyano-1'-méthyléthyl)-1,2-diméthyl-1-cyclopentyle (procédé général pour tous les isomères)

Le produit obtenu selon B. ci-dessus a été pyrolysé à 450° et $2x10^3$ Pa dans une colonne ayant une longueur de 2 m et un diamètre de 30 mm. On a ainsi obtenu un mélange qui contenait environ 30% du composé (I'a), environ 63% du composé (I'b) et des traces du composé (I'c). Le mélange obtenu peut être utilisé tel quel en tant qu'ingrédient parfumant. Cependant, il est aussi possible de séparer le composé (I'a) du composé (I'b), ainsi que séparer les isomères respectifs de chaque composé (I'a) et (I'b), par exemple par chromatographie en phase gazeuse.
Spectres des composés obtenus à partir de l'acétate de (1R,2S,3R)-3-(2'-cyano-1'-méthyléthyl)-1,2-diméthyl-1-cyclopentyle :

(1'R,2'S,3S)-3-(2'-méthyl-3'-méthylène-1'-cyclopentyl)butanenitrile (15%)

RMN($^1$H) : 0,90(d, J=8, 3H); 1,12(d, J=6, 3H); 4,8 et 4,6(deux s large, 2H) δ ppm
SM : 163($M^+$,2) : 148(4), 123(7), 107(5), 95(100), 79(13), 67(24), 41(11)

Odeur : décrite auparavant

(1'R,2'S,3R)-3-(2'-méthyl-3'-méthylène-1'-cyclopentyl)butanenitrile (15%)

RMN($^1$H) : 0,90(d, J=8, 3H); 1,14(d, J=6, 3H); 4,79 et 4,86(deux s large, 2H) δ ppm
SM : 163($M^+$,2) : 148(3), 123(4), 107(5), 95(100), 79(11), 67(21), 41(19)

Odeur : décrite auparavant

(1'R,2'S,3R)-3-(2'-3'-diméthyl-3'-cyclopentène-1'-yl)butanenitrile (29%)

RMN($^1$H) : 0,83(d, J=8, 3H); 1,09(d, J=6, 3H); 1,69(d dédoublé, 3H); 5,66(large s, 1H) δ ppm
SM : 163($M^+$,6) : 148(6), 120(8), 107(18), 95(100), 79(12), 67(16), 41(15)

(1'R,2'S,3S)-3-(2'-3'-diméthyl-3-cyclopentène-1-yl)butanenitrile (33%)

RMN($^1$H) : 0,84(d, J=8, 3H); 1,14(d, J=6, 3H); 1,70(d dédoublé, 3H); 5,24(large s, 1H) δ ppm
SM : 163($M^+$,7) : 148(6), 123(3), 107(17), 95(100), 79(11), 67(16), 41(15)

Spectres du mélange de composés obtenu à partir de l'acétate de (1R,2S,3S)-3-(2'-cyano-1'-méthyléthyl)-1,2-diméthyl-1-cyclopentyle [mélange de (1'S,2'S)-3-(2',3'-diméthyl-3'-cyclopenten-1'-yl)butanenitrile et (1'S,2'S)-(2'-méthyl-3'-méthylène-1'-cyclopentyl)butanenitrile] :

RMN($^1$H) (mélange) : 0,92-1,18(plusieurs d, 6H); 1,63 (large s, 3H); 4,78-4,86(deux s, $C=CH_2$), 5,22(large s, C=CH)
SM : 163($M^+$,12) : 148(10), 120(17), 110(15), 107(27), 95(100), 79(21), 67(27), 41(31) (non attribué)
SM : 163($M^+$,2): 148(3), 120(3), 95(100), 79(11), 67(20), 40(40) (non attribué)
SM : 163($M^+$,2) : 148(3), 120(6), 95(100), 79(13), 67(23), 41(23) (non attribué)

Le mélange était composé de quatre isomères dont deux peuvent être séparés par une chromatographie en phase gazeuse. La configuration des nitriles présents dans le mélange à été attribuée à partir de celle du cyclopentanol de départ.

Spectres du mélange obtenu à partir de l'acétate de (1R,2R,3S)-3-(2'-cyano-1'-méthyléthyl)-1,2-diméthyl-1-cyclopentyle [mélange de (1'S,2'R)-3-(2',3'-dirnéthyl-3'-cyclopenten-1'-yl)butanenitrile et (1'S,2'R)-(2'-méthyl-3'-méthylène-1'-cyclopentyl) butanenitrile] :

RMN($^1$H) (mélange) : 0,8-0,85(plusieurs d, 3H); 1,1-1,18(plusieurs d, 3H); 1,62 et 1,7(deux large s, 3H); 4,79 et 4,85(deux s, $C=CH_2$) ; 5,25(large s, C=CH)
SM : 163($M^+$,7) : 95(100), 77(7), 67(16), 41(12) (non attribué)
SM : 163($M^+$,7) : 148(7), 120(8), 107(18), 95(100), 79(13), 67(18), 41(20) (non attribué)
SM : 163($M^+$,8) : 95(100), 77(7) (non attribué)

Le mélange était composé de 4 isomères en quantités relatives de 14%, 36%, 23% et 25% (valeurs établies par chromatographie en phase gazeuse). La configuration des nitriles présents dans le mélange a été attribuée à partir de la configuration du cyclopentanol de départ.

Exemple 2

Préparation des aldéhydes de l'invention

**A.** Préparation de l'acétate de (1R,2S,3R)-3-(2-formyl-1-méthyléthyl)-1,2-diméthyl-1-cyclopentyle

Du chlorure d'acétyle (27 g; 0,34 mole) a été ajouté goutte à goutte à une solution sous agitation de (1R,2S,3S)-3-isopropényl-1,2-diméthyl-1-cyclopentanol (50 g ; 0,32 mole), anhydride acétique (20 g ; 0,2 mole), pyridine (57 g ; 0,72 mole) et toluène (600 ml) à température ambiante et chauffé à 45°C durant 3 jours. Le mélange réactionnel refroidi a été versé dans de la glace et lavé avec $H_2SO_4$ (10%), de l'eau, une solution $NaHCO_3$ (5%) et de la saumure, puis distillé à 53-56°C/0,7 hPa pour donner 59 g d'acétate de (1R,2S,3S)-3-isopropényl-1,2-diméthyl-1-cyclopentyl pur à 90%. Une distillation fractionnée a permis d'obtenir une pureté de 93% d'acétate de (1R,2S,3S)-3-isopropényl-1,2-diméthyl-1-cyclopentyle (48,7 g ; 76,6%). Ce produit présente une odeur de type lactonique avec des connotations de type jasmin et acétate de terpényle.
On a ensuite soumis le composé à une hydroformylation sous les conditions réactionnelles décrites dans l'Exemple 1, pour obtenir le composé en titre.

**B.** Pyrolyse de l'acétate de (1R,2S,3R)-3-(2-formyl-1-méthyléthyl)-1,2-diméthyl-1-cyclopentyle

Le four à thermolyse utilisé a été équipé d'un tube de quartz (longueur 1,8 m, diamètre 30 mm, rempli de granules de quartz sur 30 cm), un entonnoir à gouttes et une sortie refroidie au $CO_2$-acétone. A 25 hPa et 460°C, on a introduit 34 g de produit obtenu sous A. goutte à goutte. On a distillé le thermolisat (45°C/0,6 hPa) afin d'obtenir les aldéhydes de formule (I'') (20,9 g ; 83,7%) sous forme d'un mélange d'isomères

cis-(1'R)-3-(2'-méthyl-3'-méthylène-1'-cyclopentyl)butanal (36%),
cis-(1'R)-(2',3'-diméthyl-2'-cyclopenten-1'-yl)butanal et

cis-(1'R)-(2',3'-diméthyl-3'-cyclopenten-1'-yl)butanal.

Les isomères peuvent être séparés par chromatographie en phase gazeuse.

RMN($^1$H) : 0,83-1,02 (8d, 6H) ; 1,7(large s) ; 4,78 et 4,83(deux s); 5,23 et 5,27(deux large s) ; 9,8(m, 1H) δ ppm (mélange)
SM : 166($M^+$,6) : 151(4), 148(2), 133(9), 122(77), 107(95), 95(100), 79(37), 67(52), 41(39) (non attribué)
SM : 166($M^+$,4): 151(8), 148(10), 133(17), 122(22), 107(59), 95(100), 79(35), 67(43), 41(31) (non attribué)
SM: 166($M^+$,13) : 151(4), 148(3), 133(9), 122(76), 107(70), 95(100), 79(50), 67(62), 41(67) (non attribué)
SM : 166($M^+$,2) : 151(3), 148(4), 133(9), 122(41), 107(40), 95(100), 67(49), 41(47) (non attribué)

**C.** Préparation du (1'R,2'R)-3-(2',3'-diméthyl-1'-cyclopentyl)butanal

L'hydrogénation du produit obtenu sous B. plus haut (19,5 g) dans l'acétate d'éthyle avec 10% de catalyseur palladium/carbone, permet d'obtenir le produit identifié ci-dessus (pe 50°C/0,5 hPa), 14,8 g (75%) sous forme d'un mélange de 4 isomères en quantités relatives de 42%, 13%, 24% et 21%.

RMN($^1$H) : 0,63 et 0,66(2d, J=7Hz) ; 0,85-1,0(d); 9,78(m, 1H) δ ppm (mélange)
MS : 168($M^+$,1) : 153(3), 124(28), 109(59), 95(100), 81(19), 69(31), 55(68), 41(51) (non attribué)
SM : 168($M^+$,0) : 153(1), 124(33), 109(56), 95(92), 81(22), 69(43), 55(100), 41(68) (non attribué)
SM : 168($M^+$,0) : 124(30), 109(61), 95(100), 81(25), 69(30), 55(81), 41(59) (non attribué)
SM : 168($M^+$,0) : 153(1), 124(21), 109(48), 95(100), 81(13), 69(23), 55(65), 41(34) (non attribué)

Exemple 3

Préparation du (1'R,2'R)-3-(2',3'-diméthyl-1-cyclopentyl)butanenitrile

On a chauffé à reflux un mélange de produit obtenu dans l'Exemple 2C. (13,6g = 81 mmole), $NH_2OH.HCl$ (7,3 g ; 105 mmole) et THF (150 ml) pendant 3 h, puis on l'a refroidi et purifié dans une solution glace/$NaHCO_3$. On a ajouté du pentane et lavé la phase organique, puis distillé à environ 70°C/0,5 hPa pour donner 9,7 g *(65,5%)* de l'oxime correspondante qui contient déjà un peu de nitrile. On a ensuite chauffé ce mélange à reflux avec de l'anhydride acétique (40 ml) durant 4 h. Après le traitement habituel, on a distillé le produit à environ 50°C/6,6 hPa et obtenu 7 g (81%) du produit désiré sous forme d'un mélange de 4 isomères en quantités relatives de 36%, 23%, 11% et 20%. Les isomères peuvent être séparés par des méthodes de chromatographie en phase gazeuse.

RMN($^1$H) : 0,62 et 0,63(2d, J=7Hz) ; 0,92-1,13(8d) δ ppm (mélange)
MS : 165($M^+$, 0,1): 164(1), 150(14), 123(12), 110(69), 97(43), 70(71), 55(100), 41(66) (non attribué)
MS : 165($M^+$, 0,1) : 164(1), 150(20), 123(10), 110(19), 97(36), 70(100), 55(94), 41(58) (non attribué)
MS : 165($M^+$, 0,1) : 164(2), 150(28), 136(16), 122(19), 97(39), 70(81), 55(100), 41(62) (non attribué)
MS : 165($M^+$, 0,1) : 164(1), 150(15), 122(14), 97(22), 70(100), 55(79), 41(48) (non attribué)
MS : 165($M^+$, 0,1): 164(1), 150(8), 136(4), 122(5), 110(9), 97(12), 70(100), 55(56), 41(41) (non attribué)
MS : 165($M^+$, 0,1): 164(1), 150(7), 122(6), 97(13), 70(100), 55(52), 41(39) (non attribué)

Exemple 4

Préparation d'un parfum pour un produit de nettoyage à usages multiples

On a préparé une composition parfumante de base à caractère hespéridé pour utilisation dans un produit d'entretien, en mélangeant les ingrédients suivants :

| Ingrédients | Parties en poids |
|---|---|
| Acétate d'hexyle | 40 |
| Aldéhyde C8 à 50% * | 20 |
| Aldéhyde C9 à 10% * | 40 |

* dans le dipropylèneglycol

(suite)

| Ingrédients | Parties en poids |
|---|---|
| Aldéhyde C10 à 50% * | 70 |
| Camphre | 25 |
| Citronellol | 80 |
| Dimyrcetol ®1) | 150 |
| Hédione ®2) | 50 |
| Géraniol | 45 |
| Géranyl Nitrile 3) | 250 |
| Linalol | 120 |
| Terpènes d'orange | 570 |
| Terpinéol | 140 |
| Terpinolène | 50 |
| Total | 1650 |

* dans le dipropylèneglycol

1) mélange de 2,6-diméthyl-7-octén-2-ol et de formiate de 1,1,5-triméthyl-6-heptényl ; origine : International Flavors & Fragrances, USA

2) dihydrojasmonate de méthyle ; origine : Firmenich SA, Genève, Suisse

3) 3,7-diméthyl-2,6-octadiènenitrile ; origine : Firmenich SA, Genève, Suisse

Lorsqu'on a ajouté à cette composition de base 150 parties en poids de nitrile selon l'invention, c'est-à-dire par exemple un mélange de composés (I'a) de configuration (1'R,2'S,3S) et (1'R,2'S,3R) et de composés (I'b) de configuration (1'R,2'S,3R) et (1'R,2'S,3S), obtenu selon le procédé décrit dans l'Exemple 1, on lui a conféré un côté beaucoup plus frais et on a augmenté d'une façon prononcée la connotation verte-naturelle et zeste de citron que l'on trouve, grâce à la présence du géranyl nitrile, dans l'odeur de la composition. L'addition des composés de l'invention a le même effet que celui de l'addition de la même quantité de citral, et on obvie ainsi au problème de l'instabilité de ce dernier dans le type de milieu "produit d'entretien" (voir exemples plus bas) auquel la composition décrite est destinée.

Exemple 5

Préparation d'un parfum pour détergents en poudre

On a préparé une composition de type floral-fleur d'oranger typique d'une formulation détergent, à partir des ingrédients suivants :

| Ingrédients | Parties en poids |
|---|---|
| Acétate de linalyle | 100 |
| Acétate de styrallyle | 20 |
| Acétate de terpényle | 120 |
| Aldéhyde hexylcinnamique | 140 |
| 2-Méthyl-undécanal à 10% * | 160 |
| Anthranilate de méthyle | 30 |
| Citronellol | 210 |
| Dihydromyrcénol | 650 |
| Diphényloxyde | 10 |
| Lorysia ® 1) | 600 |
| Habanolide® 2) | 270 |
| Hélional 3) | 40 |

* dans le dipropylèneglycol

1) acétate de 4-(1,1-diméthyléthyl-1-cyclohexyl) ; origine : Firmenich SA, Genève, Suisse

2) pentadécénolide ; origine : Firmenich SA, Genève, Suisse

3) 3-(1,3-benzodioxol-5-yl)-2-méthylpropanal : origine : Firmenich SA, Genève, Suisse

(suite)

| Ingrédients | Parties en poids |
|---|---|
| Iralia® 4) | 50 |
| Essence de lavandin | 30 |
| Lilial® 5) | 100 |
| Linalol | 140 |
| Mousse cristal à 10% * | 80 |
| Acétate de 1,3-diméthyl-3-phénylbutyle à 10% * 6) | 30 |
| Nérol | 70 |
| Polysantol® 7) | 20 |
| Essence d'orange | 80 |
| 1-Méthyl-4-(4-méthyl-3-pentényl)-3-Cyclohexène carboxaldéhyde 8) | 40 |
| Salicylate d'amyle | 80 |
| 4-tert-Butyl-1-cyclohexanol 9) | 30 |
| $\alpha$-Terpinéol | 90 |
| Triplal ® 10) | 10 |
| Vertofix coeur 11) | 100 |
| Total | 3300 |

* dans le dipropylèneglycol
4) méthylionone ; origine : Firmenich SA, Genève, Suisse
5) 3-(4-tert-butylphényl)-2-méthylpropanal ; origine : Givaudan-Roure SA, Vernier, Suisse
6) origine : Firmenich SA, Genève, Suisse
7) 3,3-diméthyl-5-(2,2,3-triméthyl-3-cyclopentén-1-yl)-4-pentén-2-ol ; origine : Firmenich SA, Genève, Suisse
8) origine : Firmenich SA, Genève, Suisse
9) origine : Firmenich SA, Genève, Suisse
10) 2,4-diméthyl-3-cyclohexén-1-carboxaldéhyde ; origine : International Flavors & Fragrances, USA
11) origine : International Flavors & Fragrances, USA

Cette composition, par l'adjonction de 50 parties en poids du mélange de composés (I'a) de configuration (1'R,2'S,3S) et (1'R,2'S,3R) et de composés (I'b) de configuration (1'R,2'S,3R) et (1'R,2'R,3S), obtenu selon le procédé décrit dans l'Exemple 1 ci-dessus, prend un joli côté hespéridé-vert de type citron-limette qui apporte beaucoup de fraîcheur à la composition. L'adjonction de la même quantité de géranyl nitrile donne certes aussi un côté hespéridé-citron à cette composition, mais l'effet est cependant beaucoup plus gras et moins frais, évoquant plutôt la citronelle que le citron-vert.

Exemples comparatifs 1 à 6

On a procédé au parfumage des articles et produits mentionnés ci-après, en ajoutant aux bases appropriées non parfumées, dans les concentrations indiquées, le mélange de composés (I'a) de configuration (1'R,2'S,3S) et (1'R, 2'S,3R) et de composés (I'b) de configuration (1'R,2'S,3R) et (1'R,2'R,3S), obtenu selon le procédé décrit dans l'Exemple 1 ci-dessus, le géranyl nitrile et le citral, puis on les a mis en réserve pendant 30 jours aux températures indiquées. Les articles ainsi stockés ont ensuite été évalués à l'aveugle pour la qualité de leur odeur et, dans certains cas, la modification de la couleur.
La valeur de la qualité olfactive résultante est donnée dans le tableau ci-après par les lettres A à E ayant les significations suivantes :

A = odeur inchangée
B = odeur légèrement modifiée
C = changement assez net
D = très fort changement, devient déplaisant
E = totalement modifié, non reconnaissable

L'intensité de l'odeur est signifiée dans une échelle croissante de 1 à 10. La modification de la couleur est indiquée par les abréviations suivantes ayant pour signification :

LCo = légèrement coloré
Co = coloré

Tableau

| | | | Concentration (% en poids) | Température | | |
|---|---|---|---|---|---|---|
| | | | | 3° | 22° | 40° |
| 1 | Détergent en poudre contenant des perborates | Mélange de nitriles insaturés de l'invention | 0,2 | A8 | A8 | A7 |
| | | Citral (pur) | 0,2 | B7 | D7 | E7 |
| | | Géranyl nitrile | 0,2 | A10 | A9 | A8 |
| 2 | Détergent en poudre concentré | Mélange de nitriles insaturés de l'invention | 0.2 | A7 | A7 | B5 |
| | | Citral (pur) | 0,2 | C4 | D6 | E6 |
| | | Géranyl nitrile | 0,2 | A9 | A8 | B6 |
| 3 | Antiperspirant (roll-on) | Mélange de nitriles insaturés de l'invention | 0,5 | A10 | B10 | B8 |
| | | Citral (pur) | 0,5 | B8 | C8 | E8 |
| | | Géranyl nitrile | 0,5 | A10 | A8 | B7 |
| 4 | Antiperspirant (deo-stick) | Mélange de nitriles insaturés de l'invention | 0,5 | A10 | A10 | A9 |
| | | Citral (pur) | 0,5 | C8 | D8 | E10 |
| | | Géranyl nitrile | 0,5 | A10 | A8 | C6 |
| 5 | Antiperspirant (dry-spray) | Mélange de nitriles insaturés de l'invention | 0,8 | A10 | A8 | A7 |
| | | Citral (pur) | 0,8 | B10 | C9 | D9 |
| | | Géranyl nitrile | 0,8 | B10 | B8 | B6 |
| 6 | Savon | Mélange de nitriles insaturés de l'invention | 1,0 | A10 | A10 | A9 |
| | | Citral (pur) | 1,0 | A7 | B7/Co | C7/Co |
| | | Géranyl nitrile | 1,0 | A9 | B8 | B7/LCo |

Ces exemples montrent que le produit de l'invention est encore plus stable que le géranyl nitrile, produit connu pour sa stabilité dans les milieux agressifs. En outre, on a montré que la différence de stabilité par rapport au citral, lui-même étant un produit dont l'odeur est très prisée dans cette sorte d'application et à laquelle l'odeur du produit de la présente invention ressemble beaucoup, est très nette, le produit de la présente invention étant de loin très supérieur.

**Revendications**

1. Composé de formule

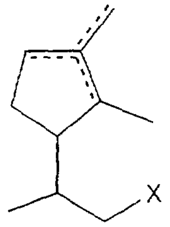

(I)

dans laquelle X représente un groupe C ≡ N ou un groupe C(H) = O et dans laquelle le cycle en $C_5$ est soit saturé, soit comprend une double liaison dans l'une des positions indiquées par les pointillés, ou tout mélange de deux ou plusieurs composés de formule (I).

2. Composé de formule (I) selon la revendication 1, dans laquelle X est un groupe C ≡ N et le cycle en $C_5$ comporte une double liaison dans l'une des positions indiquées par les pointillés, ou tout mélange de deux ou plusieurs composés de ce type.

3. Composé selon la revendication 2, **caractérisé en ce que** le composé se trouve sous forme d'un isomère de formule

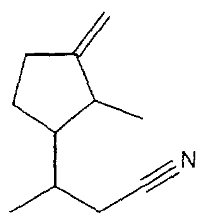

(I'a)

4. Composé selon la revendication 3, **caractérisé en ce que** le composé de formule (I'a) se présente sous forme de l'isomère de configuration (1'R,2'S,3S) ou (1'R,2'S,3R) ou d'un mélange de ces isomères.

5. Mélange selon la revendication 2, **caractérisé en ce que** ledit mélange contient environ 30% en poids du composé de formule (I'a) telle que définie à la revendication 3 et environ 60% en poids d'un composé de formule

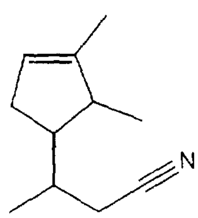

(I'b)

6. Mélange selon la revendication 5, **caractérisé en ce que** le composé de formule (I'a) se présente sous forme de l'isomère de configuration (1'R,2'S,3S) ou (1'R,2'S,3R) ou d'un mélange de ces isomères.

7. Utilisation en tant qu'ingrédient parfumant d'un composé ou d'un mélange selon l'une des revendications 1 à 6.

8. Composition parfumante ou article parfumé contenant à titre d'ingrédient parfumant un composé ou un mélange selon l'une des revendications 1 à 6.

9. Article parfumé selon la revendication 8, sous forme d'un parfum ou d'une eau de toilette, d'un savon, d'un gel de

douche ou bain, d'un shampoing ou autre produit d'hygiène capillaire, d'une préparation cosmétique, d'un déodorant corporel ou antiperspirant, d'un désodorisant d'air ambiant, d'un adoucissant textile ou d'un produit d'entretien.

**10.** Déodorant ou antiperspirant corporel, contenant à titre d'ingrédient parfumant un composé ou un mélange selon l'une des revendications 1 à 6.

**11.** Détergent contenant à titre d'ingrédient parfumant un composé ou un mélange selon l'une des revendications 1 à 6.

**12.** Procédé pour la préparation d'un composé ou d'un mélange selon l'une des revendications 2 à 6, comprenant une réaction d'élimination à l'aide d'un traitement thermique ou acide d'un ester de formule

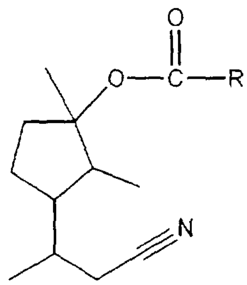

(II)

dans laquelle R est un groupe alkyle ou aryle, de préférence de l'acétate de 3-(2'-cyano-1-méthyléthyl)-1,2-diméthyl-1-cyclopentyle (R = $CH_3$) et, le cas échéant, la séparation des composés ainsi formés.

**13.** Procédé selon la revendication 12, **caractérisé en ce que** ledit traitement comporte une pyrolyse.

**14.** Procédé selon la revendication 13, **caractérisé en ce que** la pyrolyse se déroule à une température comprise entre 400 et 550°C, de préférence comprise entre 430 et 500°C, et à une pression comprise entre 10 et $50 \times 10^2$ Pa.

**15.** Procédé selon l'une des revendications 12 à 14, **caractérisé en ce que** ledit acétate possède la configuration absolue (1R,2S,3R).

**16.** Procédé pour la préparation d'un composé de formule

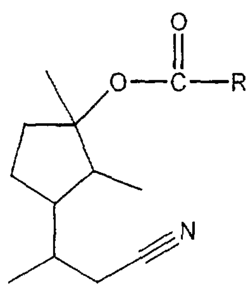

(II)

dans laquelle R est un groupe alkyle ou aryle, de préférence $CH_3$, **caractérisé en ce qu'**il comprend les étapes suivantes :

a) l'hydroformylation d'un composé de formule

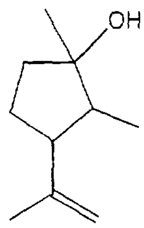

pour obtenir un aldéhyde de formule

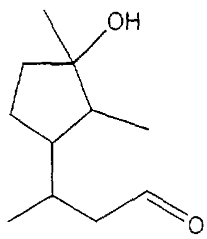

b) la transformation dudit aldéhyde à l'aide de l'hydroxylamine ou de l'un de ses sels et d'un agent d'estérification pour former le composé de formule (II).

**17.** Composé de formule

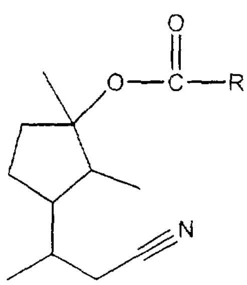

(II)

dans laquelle R est un groupe alkyle ou aryle, de préférence $CH_3$.

**Patentansprüche**

**1.** Verbindung der Formel

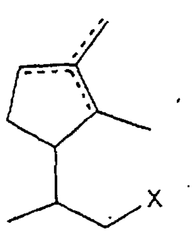

(I),

in welcher X für eine Gruppe C ≡ N oder eine Gruppe C(H) = O steht und in welcher der Ring am $C_5$ entweder gesättigt ist oder eine Doppelbindung in einer der durch die gestrichelten Linien angegebenen Positionen aufweist,

oder jegliche Mischung von zwei oder mehr Verbindungen der Formel (I).

2. Verbindung der Formel (I) nach Anspruch 1, in der X eine Gruppe C≡N ist und der Ring am $C_5$ eine Doppelbindung in einer der durch die gestrichelten Linien angegebenen Positionen aufweist, oder jegliche Mischung von zwei oder mehr Verbindungen dieses Typs.

3. Verbindung nach Anspruch 2, **dadurch gekennzeichnet, daß** die Verbindung in Form eines Isomers der Formel

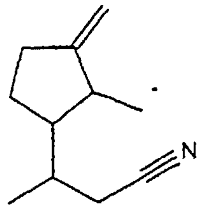

(I'a)

vorliegt.

4. Verbindung nach Anspruch 3, **dadurch gekennzeichnet, daß** die Verbindung der Formel (I'a) in Form des Isomers mit der Konfiguration (1'R,2'S,3S) oder (1'R,2'S,3R) oder einer Mischung dieser Isomere vorliegt.

5. Mischung nach Anspruch 2, **dadurch gekennzeichnet, daß** die Mischung ca. 30 Gew.-% der Verbindung der Formel (I'a) gemäß der Definition in Anspruch 3 sowie ca. 60% Gew.-% einer Verbindung der Formel

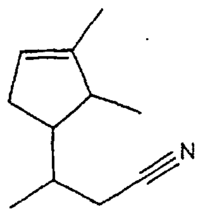

(I'b)

enthält.

6. Mischung nach Anspruch 5, **dadurch gekennzeichnet, daß** die Verbindung der Formel (I'a) in Form des Isomers mit der Konfiguration (1'R,2'S,3S) oder (1'R,2'S,3R) oder einer Mischung dieser Isomere vorliegt.

7. Verwendung einer Verbindung oder einer Mischung nach einem der Ansprüche 1 bis 6 als Riechstoff.

8. Riechstoffzusammensetzung oder parfümierter Artikel, welche bzw. welcher eine Verbindung oder eine Mischung nach einem der Ansprüche 1 bis 6 als Riechstoff enthält.

9. Parfümierter Artikel nach Anspruch 8 in Form eines Parfüms oder Eau de Toilette, einer Seife, eines Dusch- oder Badegels, eines Shampoo oder eines anderen Haarpflegeproduktes, eines kosmetischen Präparats, eines Deodorant oder Antitranspirans, eines Raumluftdeodorant, eines textilen Weichspülers oder eines Allzweckreinigers.

10. Deodorant oder Antitranspirans, welches eine Verbindung oder eine Mischung nach einem der Ansprüche 1 bis 6 als Riechstoff enthält.

11. Detergens, welches eine Verbindung oder eine Mischung nach einem der Ansprüche 1 bis 6 als Riechstoff enthält.

12. Verfahren zur Herstellung einer Verbindung oder einer Mischung nach einem der Ansprüche 2 bis 6, welches eine Abspaltungsreaktion mittels einer Wärme- oder Säurebehandlung eines Esters der Formel

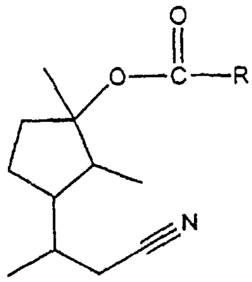

(II)

beinhaltet, in der R eine Alkyl- oder Arylgruppe ist, vorzugsweise des Acetats von 3-(2'-Cyano-1-methylethyl)-1,2-dimethyl-1-cyclopentyl($R = CH_3$), sowie gegebenenfalls die Abtrennung der hierdurch gebildeten Verbindungen.

13. Verfahren nach Anspruch 12, **dadurch gekennzeichnet, daß** die Behandlung eine Pyrolyse beinhaltet.

14. Verfahren nach Anspruch 13, **dadurch gekennzeichnet, daß** die Pyrolyse bei einer zwischen 400 und 550°C, vorzugsweise zwischen 430 und 500°C liegenden Temperatur und einem zwischen 10 und $50x10^2$ Pa liegenden Druck abläuft.

15. Verfahren nach einem der Ansprüche 12 bis 14, **dadurch gekennzeichnet, daß** das Acetat die absolute Konfiguration (1R,2S,3R) besitzt.

16. Verfahren zur Herstellung einer Verbindung der Formel

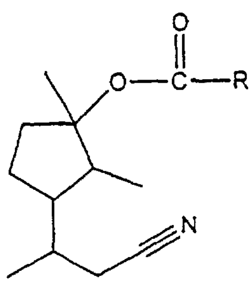

(II),

in der R eine Alkyl- oder Arylgruppe, vorzugsweise $CH_3$ ist, **dadurch gekennzeichnet, daß** es die folgenden Schritte aufweist:

a) Hydroformylierung einer Verbindung der Formel

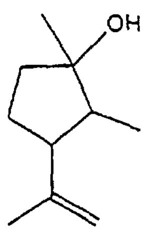

zum Herstellen eines Aldehyds der Formel

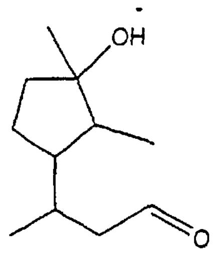

b) Umsetzung des Aldehyds mittels Hydroxylamin oder eines seiner Salze und eines Veresterungsmittels zum . Bilden der Verbindung der Formel (II).

**17.** Verbindung der Formel

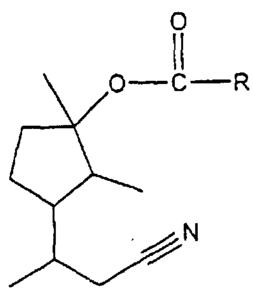

(II),

in der R eine Alkyl- oder Arylgruppe, vorzugsweise $CH_3$ ist.

## Claims

**1.** Compound of formula

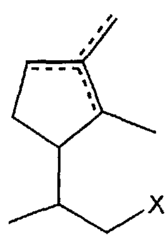

(I)

in which X is a C ≡ N group or a C(H)=O group and the $C_5$-ring is either saturated carries a double bond in one of the positions indicated by the dotted lines, or a mixture of two or more compounds of formula (I).

**2.** A compound of formula (I) according to claim 1, wherein X is a C ≡ group and the $C_5$-ring has a double bond in one of the positions indicated by the dott lines, or any mixture of 2 or more compounds of this type.

**3.** Compound according to claim 2, **characterized in that** it is in the form an isomer of formula

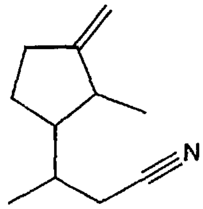

(I'a)

4. Compound according to claim 3, **characterized in that** the compound formula (I'a) is in the form of an isomer having the configuration (1'R,2'S,3S) (1'R,2'S,3R), or of a mixture thereof.

5. Mixture according to claim 2, **characterized in that** it contains about 3( by weight of the compound of formula (I'a) as defined in claim 3 and about 60% weight of its isomer of formula

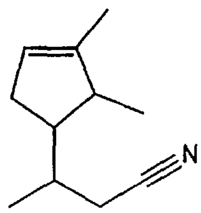

(I'b)

6. Mixture according to claim 5, **characterized in that** the compound of formula(I'a) is in the form of the isomer having the configuration (1'R,2'S,3S) or (1'R,2'S,3R), or of a mixture of these isomers.

7. Use as a perfuming ingredient of a compound or of a mixture of compounds according to anyone of claims 1 to 6.

8. Perfuming composition of perfumed article containing as perfuming ingredient a compound or a mixture of compounds according to anyone of claims 1 to 6.

9. Perfumed article according to claim 8, in the form of a perfume or cologne, a soap, a bath or shower gel, a shampoo or other hair care product, a cosmetic preparation, a body deodorant or antiperspirant, an air freshener, a fabric detergent or softener or an all-purpose household cleaner.

10. Body deodorant or antiperspirant, containing as perfuming ingredient a compound or a mixture of compounds according to anyone of claims 1 to 6.

11. Detergent containing as perfuming ingredient a compound or a mixture of compounds according to anyone of claims 1 to 6.

12. Process for the preparation of a compound or of a mixture of compounds according to anyone of claims 2 to 6, wherein an ester of formula

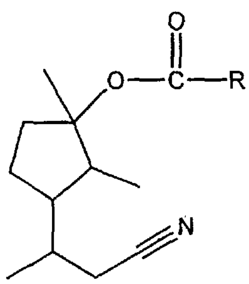

(II)

in which R is an alkyl or aryl group, and preferably 3-(2'-cyano-1'-methylethyl)-1,2-dimethyl-1-cyclopentyl acetate) ($R = CH_3$) is subjected to an elimination reaction via a thermal treatment or treatment with an acid, optionally

followed by the separation of the thus-formed compounds.

**13.** Process according to claim 12, wherein said treatment comprises a pyrolysis step.

**14.** Process according to claim 13, wherein the pyrolysis is carried out at a temperature comprised between 400 and 550°C, preferably comprised between 430 and 500°C, and a pressure comprised between 10 and $50x10^2$ Pa.

**15.** Process according to anyone of claims 12 to 14, wherein said acetate has the absolute configuration (1R,2S,3R).

**16.** Process for the preparation of a compound of formula

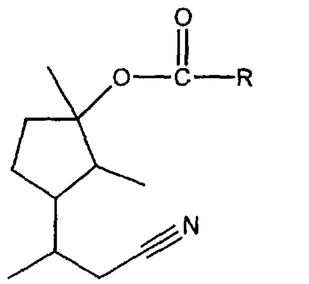

(II)

in which R is an alkyl or aryl group, and preferably $CH_3$, **characterized in that** it comprises the following steps:

a) hydroformylation of a compound of formula

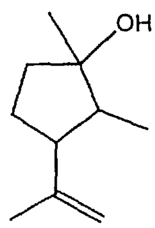

to obtain the aldehyde of formula

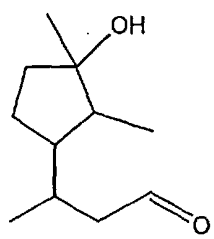

b) conversion of said aldehyde, using hydroxylamine or one of its salts and an esterifying agent, into the compound of formula (II).

**17.** Compound of formula

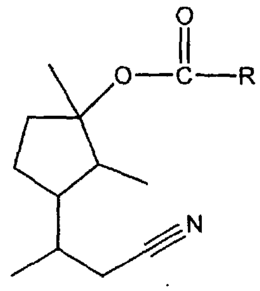

(II)

in which R is an alkyl or aryl group, and preferably $CH_3$.